# EUROPEAN PATENT SPECIFICATION

(11) **EP 3 585 189 B1**
(45) Date of publication and mention of the grant of the patent: **27.07.2022**
(21) Application number: 18703243.8
(22) Date of filing: 24.01.2018
(51) Int. Cl.: A24F 40/42, A24F 40/10, A24F 40/46, A24F 40/30

(54) **AN AEROSOL-GENERATING SYSTEM AND A CARTRIDGE FOR AN AEROSOL GENERATING SYSTEM HAVING A TWO-PART LIQUID STORAGE COMPARTMENT**
AEROSOLERZEUGUNGSSYSTEM UND KARTUSCHE FÜR EIN AEROSOLERZEUGUNGSSYSTEM MIT EINEM ZWEITEILIGEN FLÜSSIGKEITSBEHÄLTER
SYSTÈME DE GÉNÉRATION D'AÉROSOL ET CARTOUCHE POUR UN SYSTÈME DE GÉNÉRATION D'AÉROSOL AVEC UNE PORTION DE STOCKAGE LIQUIDE EN DEUX PARTIES

(30) Priority: 24.02.2017 EP 17157960
(43) Date of publication of application: 01.01.2020
(73) Proprietor: Philip Morris Products S.A., 2000 Neuchâtel (CH)
(72) Inventor: SILVESTRINI, Patrick Charles, 2000 Neuchâtel (CH); ZINOVIK, Ihar Nikolaevich, 2034 Peseux (CH); FREDERICK, Guillaume, 2206 Les-Geneveys-sur-Coffrane (CH)
(74) Representative: Ponder, William Anthony John
(86) International application number: PCT/EP2018/051730
(87) International publication number: WO 2018/153608

(56) References cited:
- WO-A1-2016/096497
- CN-U- 203 341 008
- CN-U- 203 969 207
- US-A1- 2013 228 191
- US-A1- 2016 073 692

## Description

The invention relates to an aerosol-generating system and a cartridge for an aerosol-generating system that is configured to heat a liquid aerosol-forming substrate to generate an aerosol. In particular the invention relates to a handheld aerosol-generating system configured to generate aerosol for user inhalation.

In handheld aerosol-generating systems that generate an aerosol from a liquid aerosol-forming substrate there is typically some means of transporting the liquid to the vicinity of an aerosol-generating element in order to replenish liquid that has been aerosolised by the aerosol-generating element. However it can be difficult to ensure that enough liquid is transported to the vicinity of the aerosol-generating element without oversupply of liquid, leading to leaks and inclusion of large liquid droplets in the aerosol inhaled by the user.

CN 203 969 207 U relates to an electronic cigarette with top liquid injection holes and a bottom heat exchange and emission component. The electronic cigarette with the top liquid injection holes and the bottom heat exchange and emission component comprises a cigarette holder, a connection component, a liquid storage pipe and a heat emission component.

Gravity can have a significant role to play in liquid transport within an aerosol-generating system. Typically the liquid is provided with a cartridge or cartomiser, containing both the liquid and an aerosol-generating element in the form of a heating element. The cartridge has a mouth end through which the user draws generated aerosol and a connection end opposite the mouth end which connects to a control unit containing control circuitry and a power supply. The heating element is typically located close to the connection end to allow for easy connection to the power supply, with the liquid held in a storage compartment between the heating element and the mouth end of the cartridge. During a typical use of this type of system, the highest part of the system is the mouth end of the cartridge, so that gravity acts to pull liquid down toward the heater. This can result in an oversupply of liquid at the heating element and in particular leakage of liquid into the airflow path through which aerosol is delivered to the user.

An alternative arrangement is to place the heating element in a position higher than the liquid storage compartment during typical use, and to use a capillary material to deliver liquid to the heater against the force of gravity. But this can lead to undersupply or inconsistent supply of liquid to the heating element particularly as the liquid storage compartment becomes emptier.

It would be desirable to provide an arrangement for an aerosol-generating system in which an adequate and consistent supply of liquid is provided to the aerosol-generating element even as the liquid storage compartment becomes emptier, while minimising liquid leakage, and in which complicated component such as pumps are not required.

The invention is defined in the appended independent claims, to which reference should now be made. Preferred or advantageous features of the invention are defined in dependent sub-claims. Aspects, embodiments or examples falling outside the scope of the appended independent claims are not part of the invention, and are merely included for illustrative or explanatory purposes.

In a first aspect there is provided a cartridge for an aerosol-generating system, the cartridge comprising:
a storage compartment containing a liquid aerosol-forming substrate, the storage compartment having a first portion and a second portion connected to one another so that liquid in the first portion can pass to the second portion;
an air flow passage passing between the first portion and the second portion of the storage compartment;
a fluid permeable aerosol-generating element positioned between the first portion and the second portion of the storage compartment and having a first side and a second side opposing the first side, the first side of the aerosol-generating element forming part of the airflow passage and the second side of the aerosol-generating element being in contact with liquid from the second portion of the storage compartment, so that liquid aerosol-forming substrate in the first portion of the storage compartment can reach the fluid permeable aerosol-generating element only through the second portion of the storage compartment.

The first portion and the second portion are connected to one another by a liquid channel so that liquid in the first portion can pass to the second portion through the liquid channel.

The aerosol-generating element may be a heating element. Alternatively the aerosol-generating element may be a vibrating element.

Advantageously, the first portion of the storage compartment has a larger liquid storage capacity than the second portion of the storage compartment. Advantageously, the first portion of the storage compartment is larger than the second portion of the storage compartment. In use the first portion of the storage compartment is typically positioned above the aerosol-generating element. Having the first portion of the storage compartment larger than the second portion of the storage compartment ensures that liquid is delivered from the first portion of the storage compartment to the second portion of the storage compartment, and so to the aerosol-generating element, during use, under the influence of gravity.

Advantageously, the second portion of the storage compartment contains a capillary material in contact with the second side of the aerosol-generating element. The capillary material delivers liquid aerosol-forming substrate to the aerosol-generating element against the force of gravity. By requiring the liquid aerosol forming substrate to be move against the force of gravity in use to reach the aerosol-generating element, the possibility of large droplets of the liquid entering the airflow passage is reduced.

The capillary material may be made of a material capable of guaranteeing that there is liquid aerosol-forming substrate in contact with at least a portion of the surface of the aerosol-generating element. The capillary material may extend into interstices or apertures in the aerosol-generating element. The aerosol-generating element may draw liquid aerosol-forming substrate into the interstices or apertures by capillary action.

A capillary material is a material that actively conveys liquid from one end of the material to another. The capillary material may have a fibrous or spongy structure. The capillary material preferably comprises a bundle of capillaries. For example, the capillary material may comprise a plurality of fibres or threads or other fine bore tubes. The fibres or threads may be generally aligned to convey liquid aerosol-forming substrate towards the aerosol-generating element. Alternatively, the capillary material may comprise sponge-like or foam-like material. The structure of the capillary material forms a plurality of small bores or tubes, through which the liquid aerosol-forming substrate can be transported by capillary action. The capillary material may comprise any suitable material or combination of materials. Examples of suitable materials are a sponge or foam material, ceramic- or graphite-based materials in the form of fibres or sintered powders, foamed metal or plastics material, a fibrous material, for example made of spun or extruded fibres, such as cellulose acetate, polyester, or bonded polyolefin, polyethylene, terylene or polypropylene fibres, nylon fibres or ceramic. The capillary material may have any suitable capillarity and porosity so as to be used with different liquid physical properties. The liquid aerosol-forming substrate has physical properties, including but not limited to viscosity, surface tension, density, thermal conductivity, boiling point and vapour pressure, which allow the liquid aerosol-forming substrate to be transported through the capillary medium by capillary action.

Alternatively, or in addition, the storage compartment may contain a carrier material for holding a liquid aerosol-forming substrate. The carrier material may be in the first portion of the storage compartment, the second portion of the storage compartment or both the first and second portions of the storage compartment. The carrier material may be a foam, and sponge of collection of fibres. The carrier material may be formed from a polymer or copolymer. In one embodiment, the carrier material is a spun polymer. The aerosol-forming substrate may be released into the carrier material during use. For example, the liquid aerosol-forming substrate may be provided in a capsule.

The cartridge may comprise a housing having a connection end and a mouth end remote from the connection end, the connection end configured to connect to a control body of an aerosol-generating system. The second side of the aerosol-generating element may face the connection end and the first side of the aerosol-generating element may face the mouth end. Electrical power may be delivered to the aerosol-generating element from a connected control body through the connection end of the housing.

Advantageously, the aerosol-generating element is closer to the connection end than to the mouth end opening. This allows for a simple and short electrical connection path between a power source in the control body and the aerosol-generating element.

The air flow passage extends between the first and second portions of the storage compartment. Additionally, the air flow passage may extend through the first portion of the storage compartment. For example, the first portion of the storage compartment may have an annular cross section, with the air flow passage extending from the aerosol-generating element to a mouth end opening through the first portion of the storage compartment. Alternatively, the air flow passage may extend from the aerosol-generating element to the mouth end opening adjacent to the first portion of the storage compartment.

The first and second sides of the aerosol-generating element may be substantially planar. The aerosol-generating element may be a heating element. The heating element may comprise a substantially flat heating element to allow for simple manufacture. Geometrically, the term "substantially flat" heating element is used to refer to a heating element that is in the form of a substantially two dimensional topological manifold. Thus, the substantially flat heating element extends in two dimensions along a surface substantially more than in a third dimension. In particular, the dimensions of the substantially flat heating element in the two dimensions within the surface is at least five times larger than in the third dimension, normal to the surface. An example of a substantially flat heating element is a structure between two substantially imaginary parallel surfaces, wherein the distance between these two imaginary surfaces is substantially smaller than the extension within the surfaces. In some embodiments, the substantially flat heating element is planar. In other embodiments, the substantially flat heating element is curved along one or more dimensions, for example forming a dome shape or bridge shape.

The heating element may comprise a plurality of interstices or apertures extending from the second side to the first side and through which fluid may pass.

The aerosol-generating element may alternatively, or in addition, comprise a vibrating membrane or mesh. A piezoelectric element may be used to vibrate the membrane or mesh. Liquid aerosol-forming substrate passing through apertures or interstices in the vibrating membrane or mesh may form droplets that form an aerosol in an airflow through the system. The aerosol-generating element may alternatively, or in addition, comprise a plurality of droplet ejectors, such as bubble jet nozzles that may use a piezoelectric or thermal ejection mechansim.

The heating element may comprise a plurality of electrically conductive filaments. The term "filament" is used throughout the specification to refer to an electrical path arranged between two electrical contacts. A filament may arbitrarily branch off and diverge into several paths or filaments, respectively, or may converge from several electrical paths into one path. A filament may have a round, square, flat or any other form of cross-section. A filament may be arranged in a straight or curved manner.

The heating element may be an array of filaments, for example arranged parallel to each other. Preferably, the filaments may form a mesh. The mesh may be woven or nonwoven. The mesh may be formed using different types of weave or lattice structures. Alternatively, the electrically conductive heating element consists of an array of filaments or a fabric of filaments. The mesh, array or fabric of electrically conductive filaments may also be characterized by its ability to retain liquid.

In a preferred embodiment, a substantially flat heating element may be constructed from a wire that is formed into a wire mesh. Preferably, the mesh has a plain weave design. Preferably, the heating element is a wire grill made from a mesh strip.

The electrically conductive filaments may define interstices between the filaments and the interstices may have a width of between 10 micrometres and 100 micrometres. Preferably, the filaments give rise to capillary action in the interstices, so that in use, liquid to be vaporized is drawn into the interstices, increasing the contact area between the heating element and the liquid aerosol-forming substrate.

The electrically conductive filaments may form a mesh of size between 60 and 240 filaments per centimetre (+/- 10 percent). Preferably, the mesh density is between 100 and 140 filaments per centimetres (+/- 10 percent). More preferably, the mesh density is approximately 115 filaments per centimetre. The width of the interstices may be between 100 micrometres and 25 micrometres, preferably between 80 micrometres and 70 micrometres, more preferably approximately 74 micrometres. The percentage of open area of the mesh, which is the ratio of the area of the interstices to the total area of the mesh may be between 40 percent and 90 percent, preferably between 85 percent and 80 percent, more preferably approximately 82 percent.

The electrically conductive filaments may have a diameter of between 8 micrometres and 100 micrometres, preferably between 10 micrometres and 50 micrometres, more preferably between 12 micrometres and 25 micrometres, and most preferably approximately 16 micrometres. The filaments may have a round cross section or may have a flattened cross-section.

The area of the mesh, array or fabric of electrically conductive filaments may be small, for example less than or equal to 50 square millimetres, preferably less than or equal to 25 square millimetres, more preferably approximately 15 square millimetres. The size is chosen such to incorporate the heating element into a handheld system. Sizing of the mesh, array or fabric of electrically conductive filaments less or equal than 50 square millimetres reduces the amount of total power required to heat the mesh, array or fabric of electrically conductive filaments while still ensuring sufficient contact of the mesh, array or fabric of electrically conductive filaments to the liquid aerosol-forming substrate. The mesh, array or fabric of electrically conductive filaments may, for example, be rectangular and have a length between 2 millimetres to 10 millimetres and a width between 2 millimetres and 10 millimetres. Preferably, the mesh has dimensions of approximately 5 millimetres by 3 millimetres.

The filaments of the heating element may be formed from any material with suitable electrical properties. Suitable materials include but are not limited to: semiconductors such as doped ceramics, electrically "conductive" ceramics (such as, for example, molybdenum disilicide), carbon, graphite, metals, metal alloys and composite materials made of a ceramic material and a metallic material. Such composite materials may comprise doped or undoped ceramics. Examples of suitable doped ceramics include doped silicon carbides. Examples of suitable metals include titanium, zirconium, tantalum and metals from the platinum group.

Examples of suitable metal alloys include stainless steel, constantan, nickel-, cobalt-, chromium-, aluminum-, titanium-, zirconium-, hafnium-, niobium-, molybdenum-, tantalum-, tungsten-, tin-, gallium-, manganese- and iron-containing alloys, and super-alloys based on nickel, iron, cobalt, stainless steel, Timetal^{®}, iron-aluminum based alloys and iron-manganese-aluminum based alloys. Timetal^{®} is a registered trade mark of Titanium Metals Corporation. The filaments may be coated with one or more insulators. Preferred materials for the electrically conductive filaments are stainless steel and graphite, more preferably 300 series stainless steel like AISI 304, 316, 304L, 316L. Additionally, the electrically conductive heating element may comprise combinations of the above materials. A combination of materials may be used to improve the control of the resistance of the substantially flat heating element. For example, materials with a high intrinsic resistance may be combined with materials with a low intrinsic resistance. This may be advantageous if one of the materials is more beneficial from other perspectives, for example price, machinability or other physical and chemical parameters. Advantageously, a substantially flat filament arrangement with increased resistance reduces parasitic losses. Advantageously, high resistivity heaters allow more efficient use of battery energy.

Preferably, the filaments are made of wire. More preferably, the wire is made of metal, most preferably made of stainless steel.

The electrical resistance of the mesh, array or fabric of electrically conductive filaments of the heating element may be between 0.3 Ohms and 4 Ohms. Preferably, the electrical resistance is equal or greater than 0.5 Ohms. More preferably, the electrical resistance of the mesh, array or fabric of electrically conductive filaments is between 0.6 Ohms and 0.8 Ohms, and most preferably about 0.68 Ohms. The electrical resistance of the mesh, array or fabric of electrically conductive filaments is preferably at least an order of magnitude, and more preferably at least two orders of magnitude, greater than the electrical resistance of electrically conductive contact areas. This ensures that the heat generated by passing current through the heating element is localized to the mesh or array of electrically conductive filaments. It is advantageous to have a low overall resistance for the heating element if the system is powered by a battery. A low resistance, high current system allows for the delivery of high power to the heating element. This allows the heating element to heat the electrically conductive filaments to a desired temperature quickly.

Alternatively, the heating element may comprise a heating plate in which an array of apertures is formed. The apertures may be formed by etching or machining, for example. The plate may be formed from any material with suitable electrical properties, such as the materials described above in relation to filaments of a heating element.

The first side of the aerosol-generating element may directly face the mouth end opening. This orientation of a planar aerosol-generating element allows for simple assembly of the cartridge during manufacture.

The heating element may be part of a heater assembly. The heater assembly may comprise the heating element and electrical contact portions, electrically connected to the heating element. The electrical contact portions may be two electrically conductive contact pads. The electrically conductive contact pads may be positioned at an edge area of the heating element. Preferably, the at least two electrically conductive contact pads may be positioned on extremities of the heating element. An electrically conductive contact pad may be fixed directly to electrically conductive filaments of the heating element. An electrically conductive contact pad may comprise a tin patch. Alternatively, an electrically conductive contact pad may be integral with the heating element.

The contact portions may exposed through a connection end of the housing to allow for contact with electrical contact pins in a control body of an aerosol-generating system.

The storage compartment may comprise a storage compartment housing. The aerosol-generating element may be fixed to the storage compartment housing. The storage compartment housing may comprise a moulded component or heater mount, the moulded component or heater mount being moulded over the heater assembly or other form of aerosol-generating element. The moulded portion or heater mount may cover a portion of the first side of the heater assembly or aerosol-generating element to isolate electrical contact portions from the airflow passage and may cover at least a portion of the second side of the heater assembly or aerosol-generating element to isolate the electrical contact portions from the liquid aerosol-forming substrate.

The heater mount may comprise at least one wall extending from the second side of the heater assembly, the at least one wall forming part of the second portion of the liquid storage compartment. The heater mount may define a liquid flow path from a first side of the heater assembly to a second side of the heater assembly.

The liquid storage compartment may hold liquid aerosol-forming substrate. As used herein with reference to the present invention, an aerosol-forming substrate is a substrate capable of releasing volatile compounds that can form an aerosol. Volatile compounds may be released by heating the aerosol-forming substrate. Volatile compounds may be released by moving the aerosol-forming substrate through passages of a vibratable element.

The aerosol-forming substrate may be liquid at room temperature. The aerosol-forming substrate may comprise both liquid and solid components. The liquid aerosol-forming substrate may comprise nicotine. The nicotine containing liquid aerosol-forming substrate may be a nicotine salt matrix. The liquid aerosol-forming substrate may comprise plant-based material. The liquid aerosol-forming substrate may comprise tobacco. The liquid aerosol-forming substrate may comprise a tobacco-containing material containing volatile tobacco flavour compounds, which are released from the aerosol-forming substrate upon heating. The liquid aerosol-forming substrate may comprise homogenised tobacco material. The liquid aerosol-forming substrate may comprise a non-tobacco-containing material. The liquid aerosol-forming substrate may comprise homogenised plant-based material.

The liquid aerosol-forming substrate may comprise one or more aerosol-formers. An aerosol-former is any suitable known compound or mixture of compounds that, in use, facilitates formation of a dense and stable aerosol and that is substantially resistant to thermal degradation at the temperature of operation of the system. Examples of suitable aerosol formers include glycerine and propylene glycol. Suitable aerosol-formers are well known in the art and include, but are not limited to: polyhydric alcohols, such as triethylene glycol, 1,3-butanediol and glycerine; esters of polyhydric alcohols, such as glycerol mono-, di- or triacetate; and aliphatic esters of mono-, di- or polycarboxylic acids, such as dimethyl dodecanedioate and dimethyl tetradecanedioate. The liquid aerosol-forming substrate may comprise water, solvents, ethanol, plant extracts and natural or artificial flavours.

The liquid aerosol-forming substrate may comprise nicotine and at least one aerosol former. The aerosol former may be glycerine or propylene glycol. The aerosol former may comprise both glycerine and propylene glycol. The liquid aerosol-forming substrate may have a nicotine concentration of between about 0.5% and about 10%, for example about 2%.

The housing may be formed form a mouldable plastics material, such as polypropylene (PP) or polyethylene terephthalate (PET). The housing may form a part or all of a wall of the storage compartment. The housing and storage compartment may be integrally formed. Alternatively the storage compartment may be formed separately from the housing and assembled to the housing.

The cartridge may comprise a removable mouthpiece through which aerosol may be drawn by a user. The removable mouthpiece may cover the mouth end opening. Alternatively the cartridge may be configured to allow a user to draw directly on the mouth end opening.

The cartridge may be refillable with liquid aerosol-forming substrate. Alternatively, the cartridge may be designed to be disposed of when the storage compartment becomes empty of liquid aerosol-forming substrate.

In a second aspect, there is provided a cartridge for an aerosol-generating system, the cartridge comprising:
a housing having a mouth end opening;
a storage compartment within the housing and containing a liquid aerosol-forming substrate;
a fluid permeable aerosol-generating element within the housing and having a first side and a second side opposing the first side, the first side of the aerosol-generating element being closer to the mouth end opening than the second side of the aerosol-generating element, and the second side of the aerosol-generating element being in contact with liquid from the storage compartment; and
an air flow passage extending from the first side of the aerosol-generating element to the mouth end opening;
wherein a first portion of the storage compartment is positioned between the aerosol-generating element and the mouth end opening and a second portion of the storage compartment is positioned on an opposite side of the aerosol-generating element to the mouth end opening.

In this context "positioned between" means that a straight line from the aerosol-generating element to the mouth end opening must pass through or past the first portion of the storage compartment. The air flow passage may pass through the storage portion.

Features of the cartridge of the first aspect may be applied to the second aspect.

In a third aspect there is provided an aerosol-generating system comprising a cartridge in accordance with the first or second aspect and a control body connected to the cartridge, the control body configured to control a supply of electrical power to the aerosol-generating element.

The control body may comprise at least one electrical contact element configured to provide an electrical connection to the aerosol-generating element when the control body is connected to the cartridge. The electrical contact element may be elongate. The electrical contact element may be spring-loaded. The electrical contact element may contact an electrical contact pad in the cartridge.

The control body may comprise a connecting portion for engagement with the connection end of the cartridge.

The control body may comprise a power supply.

The control body may comprise control circuitry configured to control a supply of power from the power supply to the aerosol-generating element.

The control circuitry may comprise a microcontroller. The microcontroller is preferably a programmable microcontroller. The control circuitry may comprise further electronic components. The control circuitry may be configured to regulate a supply of power to the aerosol-generating element. Power may be supplied to the aerosol-generating element continuously following activation of the system or may be supplied intermittently, such as on a puff-by-puff basis. The power may be supplied to the aerosol-generating element in the form of pulses of electrical current.

The control body may comprise a power supply arranged to supply power to at least one of the control system and the aerosol-generating element. The aerosol-generating element may comprise an independent power supply. The aerosol-generating system may comprise a first power supply arranged to supply power to the control circuitry and a second power supply configured to supply power to the aerosol-generating element.

The power supply may be a DC power supply. The power supply may be a battery. The battery may be a Lithium based battery, for example a Lithium-Cobalt, a Lithium-Iron-Phosphate, a Lithium Titanate or a Lithium-Polymer battery. The battery may be a Nickel-metal hydride battery or a Nickel cadmium battery. The power supply may be another form of charge storage device such as a capacitor. The power supply may require recharging and be configured for many cycles of charge and discharge. The power supply may have a capacity that allows for the storage of enough energy for one or more user experiences; for example, the power supply may have sufficient capacity to allow for the continuous generation of aerosol for a period of around six minutes, corresponding to the typical time taken to smoke a conventional cigarette, or for a period that is a multiple of six minutes. In another example, the power supply may have sufficient capacity to allow for a predetermined number of puffs or discrete activations of the atomising assembly.

The aerosol-generating system may be a handheld aerosol-generating system. The handheld aerosol-generating system may be configured to allow a user to suck on a mouthpiece to draw an aerosol through the mouth end opening. The aerosol-generating system may have a size comparable to a conventional cigar or cigarette. The aerosol-generating system may have a total length between about 30 mm and about 150 mm. The aerosol-generating system may have an external diameter between about 5 mm and about 30mm.

Although the system of the invention has been described as comprising a cartridge and a control body, it is possible to implement the invention in a one-piece system. In a fourth aspect, there is provided an aerosol-generating system comprising:
a housing having an air inlet and mouth end opening;
a storage compartment within the housing containing a liquid aerosol-forming substrate, the storage compartment having a first portion and a second portion connected to one another so that liquid in the first portion can pass to the second portion;
an air flow passage passing from the air inlet between the first portion and the second portion of the storage compartment to the mouth end opening;
a fluid permeable aerosol-generating element positioned between the first portion and the second portion of the storage compartment and having a first side and a second side opposing the first side, the first side of the aerosol-generating element forming part of the airflow passage and the second side of the aerosol-generating element being in contact with liquid from the second portion of the storage compartment, so that liquid aerosol-forming substrate in the first portion of the storage compartment must pass to the second portion of the storage compartment to reach the fluid permeable aerosol-generating element.
a power supply within the housing connected to the aerosol-generating element; and
control circuitry within the housing and configured to control a supply of power from the power supply to the aerosol-generating element.

In a fifth aspect, there is provided an aerosol-generating system comprising:
a housing having a mouth end opening;
a storage compartment within the housing and containing a liquid aerosol-forming substrate;
a fluid permeable aerosol-generating element within the housing and having a first side and a second side opposing the first side, the first side of the aerosol-generating element being closer to the mouth end opening than the second side of the aerosol-generating element, and the second side of the aerosol-generating element being in contact with liquid from the storage compartment;
an air flow passage extending from the first side of the aerosol-generating element to the mouth end opening;
a power supply within the housing connected to the aerosol-generating element; and
control circuitry within the housing and configured to control a supply of power from the power supply to the aerosol-generating element;
wherein a first portion of the storage compartment is positioned between the aerosol-generating element and the mouth end opening and a second portion of the storage compartment is positioned on an opposite side of the aerosol-generating element to the mouth end opening.

The aerosol-genarating element may be generally planar. The second side of the aerosol-generating element may be positioned outside of the air flow passage. The second side of the aerosol-generating element may be in contact with the liquid from the storage compartment but not in contact with air in the air flow passage.

The first portion of the storage compartment and the second portion of the storage compartment are connected to one another by a liquid channel so that liquid in the first portion can pass to the second portion through the liquid channel.The aerosol-generating element of the fourth and fifth aspects may comprise any of the features of the aerosol-generating element described in relation to the first and second aspects.

The storage compartment of the fourth and fifth aspects may comprise any of the features of the storage compartment described in relation to the first and second aspects. The storage compartment may be refillable with liquid aerosol-forming substrate. Alternatively, the system may be designed to be disposed of when the storage compartment becomes empty of liquid aerosol-forming substrate.

In the fourth and fifth aspects, the housing may be elongate. The housing may comprise any suitable material or combination of materials. Examples of suitable materials include metals, alloys, plastics or composite materials containing one or more of those materials, or thermoplastics that are suitable for food or pharmaceutical applications, for example polypropylene, polyetheretherketone (PEEK) and polyethylene. The material may be light and non-brittle. The housing may comprise any of the features of the housing described in relation to the first aspect.

The air flow passage of the fourth and fifth aspects may comprise any of the features of the air flow passage described in relation to the first and second aspects.

The power supply of the fourth and fifth aspects may comprise any of the features of the power supply described in relation to the third aspect.

The control circuitry of the fourth and fifth aspects may comprise any of the features of the control circuitry described in relation to the third aspect.

The cartridge, control body or aerosol-generating system in any of the aspects may comprise a puff detector in communication with the control circuitry. The puff detector may be configured to detect when a user draws through the airflow passage.

The cartridge, control body or aerosol-generating system in any of the aspects may comprise a temperature sensor in communication with the control circuitry. The cartridge, control body or aerosol-generating system may comprise a user input, such as a switch or button. The user input may enable a user to turn the system on and off.

The cartridge, control body or aerosol-generating system may also comprise indication means for indicating the determined amount of liquid aerosol-forming substrate held in the liquid storage portion to a user. The control circuitry may be configured to activate the indication means after a determination of the amount of liquid aerosol-forming substrate held in the liquid storage portion has been made.

The indication means may comprise one or more of lights, such as light emitting diodes (LEDs), a display, such as an LCD display and audible indication means, such as a loudspeaker or buzzer and vibrating means. The control circuitry may be configured to light one or more of the lights, display an amount on the display, emit sounds via the loudspeaker or buzzer and vibrate the vibrating means.

Features of one aspect may be applied to the other aspects.

Embodiments of the invention will now be described in detail, by way of example only, with reference to the accompanying drawings, in which:
Figure 1 is a schematic illustration of an aerosol-generating system in accordance with the invention;
Figure 2a is a schematic illustration of a first cross-section of a cartridge, including a mouthpiece, in accordance with the invention;
Figure 2b is a schematic illustration of a second cross-section of a cartridge in accordance with the invention;
Figure 3 illustrates a cartridge without a mouthpiece;
Figures 4a and 4b illustrate the heater mount of Figure 3a and 2b and of Figure 3;
Figures 5a and 5b are top perspective views of the heater assembly and heater mount of Figures 4a and 4b;
Figures 6a and 6b are bottom views of the heater assembly and heater mount of Figures 4a and 4b; and
Figure 7 illustrates the electrical connection of a control body to the heater assembly.

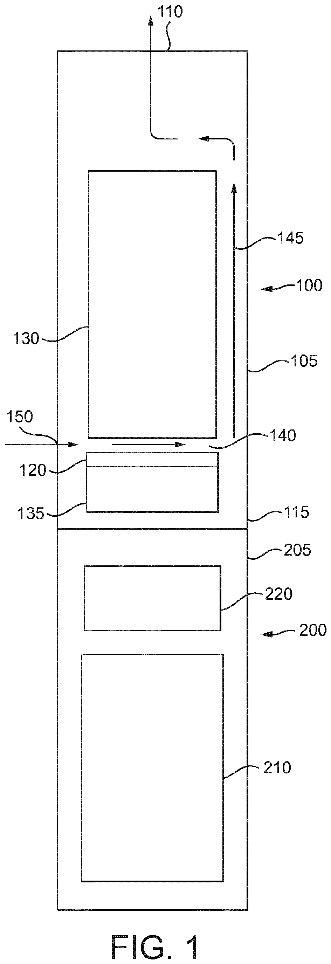

Figure 1 is a schematic illustration of an aerosol-generating system in accordance with the invention. The system comprises two main components, a cartridge 100 and a control body 200. A connection end 115 of the cartridge 100 is removably connected to a corresponding connection end 205 of the control body 200. The control body 200 contains a battery 210, which in this example is a rechargeable lithium ion battery, and control circuitry 220. The aerosol-generating device 10 is portable and has a size comparable to a conventional cigar or cigarette.

The cartridge 100 comprises a housing 105 containing an atomising assembly 120 and a liquid storage compartment having a first portion 130 and a second portion 135. A liquid aerosol-forming substrate is held in the liquid storage compartment. Although not illustrated in Figure 1, the first portion 130 of the liquid storage compartment is connected to the second portion135 of the liquid storage compartment so that liquid in the first portion 130 can pass to the second portion 135. The atomising assembly 120 receives liquid from the second portion 135 of the liquid storage compartment. In this embodiment, the atomising assembly 120 is a generally planar, fluid permeable heater assembly.

An air flow passage 140, 145 extends through the cartridge 100 from an air inlet 150 past the atomising assembly 120 and from the atomising assembly 120 to a mouth end opening 110 in the cartridge housing 105.

The components of the cartridge 100 are arranged so that the first portion 130 of the liquid storage compartment is between the atomising assembly 120 and the mouth end opening 110, and the second portion 135 of the liquid storage compartment is positioned on an opposite side of the atomising assembly 120 to the mouth end opening 110. In other words, the atomising assembly 120 lies between the two portions 130, 135 of the liquid storage compartment and receives liquid from the second portion 135, and the first portion 130 of liquid storage compartment is closer to the mouth end opening 110 than the second portion 135 of the liquid storage compartment. The air flow passage extends past the atomising assembly 120 and between the first and second portion 130, 135 of the liquid storage compartment.

The system is configured so that a user can puff or suck on the mouth end opening 110 of the cartridge 100 to draw aerosol into their mouth. In operation, when a user puffs on the mouth end opening 110, air is drawn through the airflow passage from the air inlet 150, past the atomising assembly 120, to the mouth end opening 110. The control circuitry 220 controls the supply of electrical power from the battery 210 to the cartridge 100 when the system is activated. This in turn controls the amount and properties of the vapour produced by the atomising assembly 120. The control circuitry 220 may include an airflow sensor and the control circuitry may 220 supply electrical power to the atomising assembly 120 when user puffs on the cartridge 100 are detected by the airflow sensor. This type of control arrangement is well established in aerosol-generating systems such as inhalers and e-cigarettes. So when a user sucks on the mouth end opening 110 of the cartridge 100, the atomising assembly 120 is activated and generates a vapour that is entrained in the air flow passing through the air flow passage 140. The vapour cools with in the airflow in passage 145 to form an aerosol, which is then drawn into the user's mouth through the mouth end opening 110.

In operation, the mouth end opening 110 is typically the highest point of the device. The construction of the cartridge 100, and in particular the arrangement of the atomising assembly 120 between first and second portions 130, 135 of the liquid storage compartment, is advantageous because it exploits gravity to ensure that the liquid substrate is delivered to the atomising assembly 120 even as the liquid storage compartment is becoming empty, but prevents an oversupply of liquid to the atomising assembly 120 which might lead to leakage of liquid into the air flow passage 140.

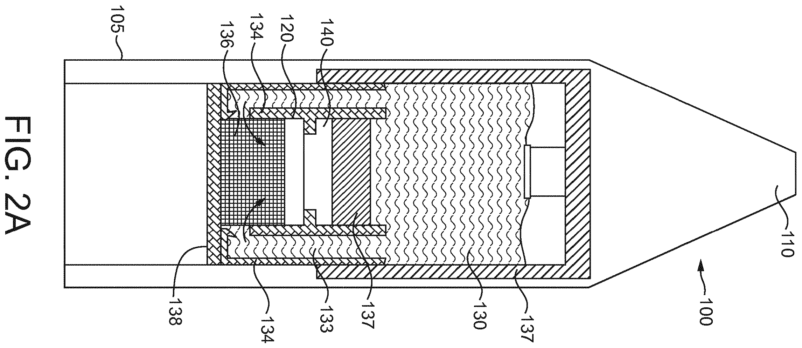

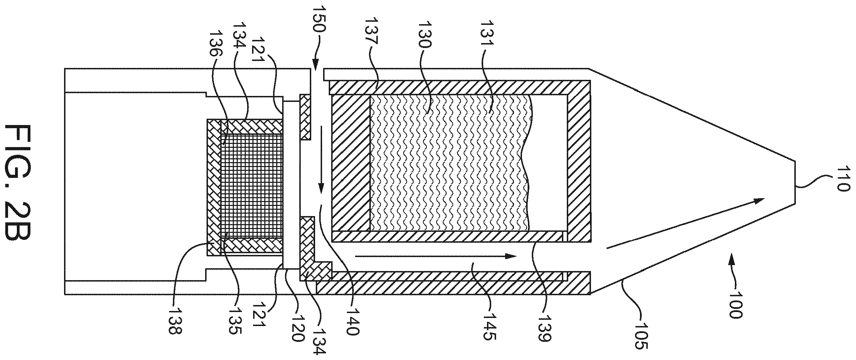

Figure 2a is a first cross section of a cartridge in accordance with an embodiment of the invention. Figure 2b is a second cross section, orthogonal to the cross section of Figure 2a.

The cartridge 100 of Figures 2a comprises an external housing 105 having a mouth end with a mouth end opening 110, and a connection end opposite the mouth end. Within the housing 105 is the liquid storage compartment holding a liquid aerosol-forming substrate 131. The liquid is contained in the liquid storage compartment by three components, an upper storage compartment housing 137, a heater mount 134 and an end cap 138. A heater assembly 120 is held in the heater mount 134. A capillary material 136 is provided in the second portion 135 of the liquid storage compartment, and abuts the heater element in a central region of the heater assembly 120. The capillary material is oriented to transport liquid to the heater element. The heater element 121 comprises a mesh heater element, formed from a plurality of filaments. Details of this type of heater element construction can be found in WO2015/117702 for example. An airflow passage 140 extends between the first and second portions 130, 135 of the liquid storage compartment. A bottom wall of the airflow passage 140 comprises the heater element 121 and the heater mount 134, side walls of the airflow passage 140 comprise portions of the heater mount 134, and a top wall of the airflow passage 140 comprises a portion of the upper storage compartment housing 137. The air flow passage 130 has a vertical portion 145 that extends through the first portion 130 of the liquid storage compartment, as shown in Figure 2a, towards the mouth end opening 110.

The heater assembly 20 is generally planar and has two faces. A first face of the heater assembly 120 faces the first portion 130 of the liquid storage compartment and the mouth end opening 110. A second face of the heater assembly 120 is in contact with the capillary material 136 and the liquid 131 in the liquid storage compartment, and faces a connection end 115 of the cartridge 100. The heater assembly 120 is closer to the connection end so that electrical connection of the heater assembly 120 to a power supply can be easily and robustly achieved, as will be described. The first portion 130 of the liquid storage compartment is larger than the second portion 135 of the liquid storage compartment and occupies a space between the heater assembly 120 and the mouth end opening 110 of the cartridge 100. Liquid in the first portion 130 of the liquid storage compartment can travel to the second portion 135 of the liquid storage compartment through liquid channels 133 on either side of the heater assembly 120. Two channels are provided in this example to provide a symmetric structure, although only one channel is necessary. The channels are enclosed liquid flow paths defined between the upper storage compartment housing 137 and the heater mount 134.

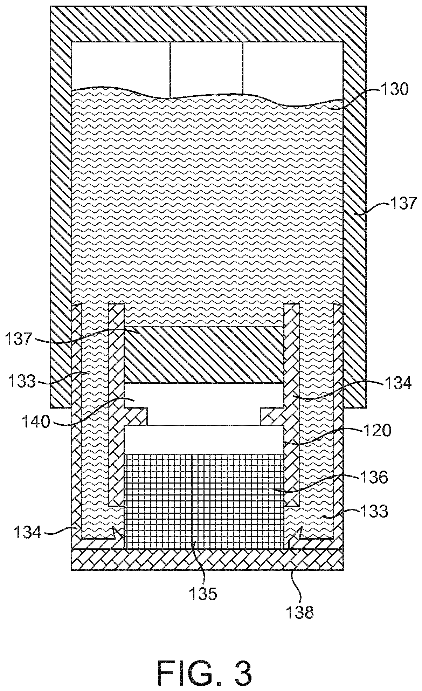

Figure 3 is an enlarged view of the liquid storage compartment and heater assembly 120 of the cartridge 100 shown in Figures 2a and 2b. It is possible to provide a cartridge comprising the components shown in Figure 3, without an external housing 105 or mouthpiece. A mouthpiece may be provided as a separate component to the cartridge 100 or maybe provided as part of the control body 200, with a cartridge as shown in Figure 3 configured to be inserted into the control body 200.

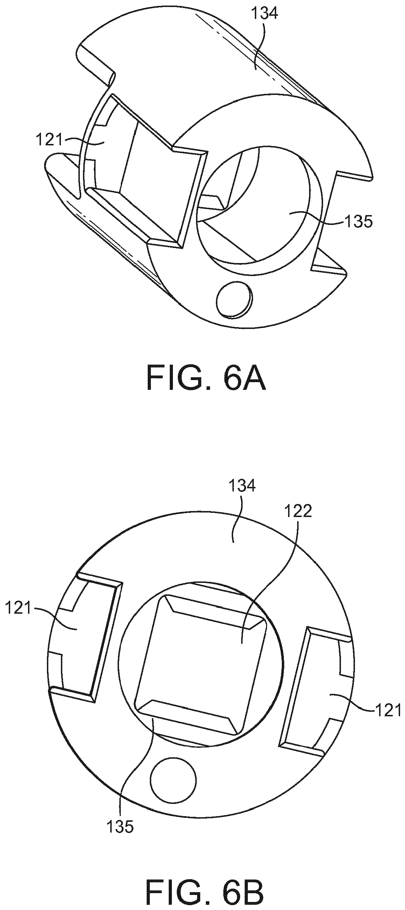

The cartridge 100 shown in Figure 3 may be assembled by first moulding the heater mount 134 around the heater assembly 120. The heater assembly comprises a mesh heater element 122 as described, fixed to a pair of tin contact pads 121, which have a much lower electrical resistivity than the heater element 122. The contact pads 121 are fixed to opposite ends of the heater element 122, as illustrated in Figures 6a and 6b. The heater mount 134 is then fixed to the upper storage portion housing using welding or adhesive . The capillary material 136 is inserted into the second portion 135 of the liquid storage compartment. The end cap 138 is then fixed to the heater mount to seal the liquid storage compartment.

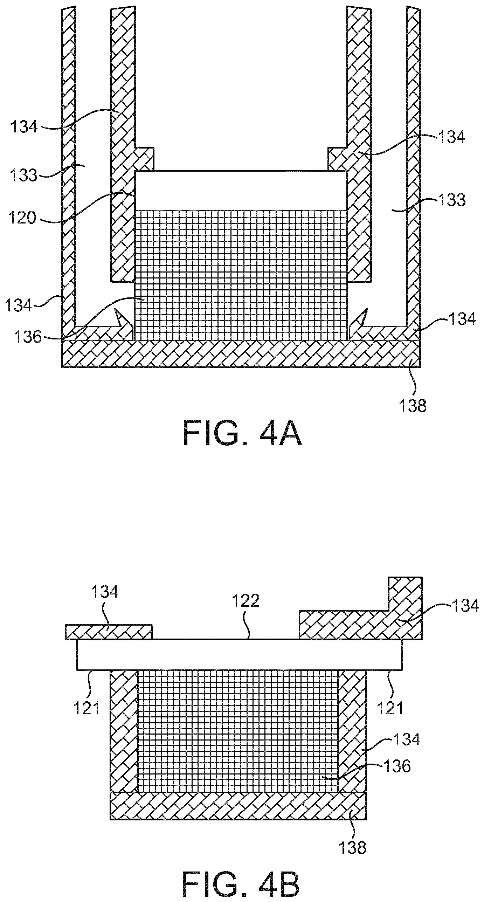

Alternatively, the heater mount capillary material 136 and end cap 138 can be assembled first before being fixed to the upper storage portion housing. Figure 4a is a first cross section of the heater assembly 120, heater mount 134, capillary material 136 and end cap 138. The liquid channels 133 are clearly shown. Figure 4b is a second cross section of the heater assembly 120, heater mount 134, capillary material 136 and end cap 138. It can be seen that the heater mount 134 secures the heater assembly 120 on both sides of the heater assembly 120. The contact pads 121 are easily accessible from the second side of the heater assembly 120 but are covered by the heater mount on the first side of the heater assembly 120 to protect them from vapour in the air flow passage 140. A lower wall of the heater mount 134 extends from the second side of the heater assembly 120 and isolates the contact pads 121 from the liquid in the second portion 135 of the liquid storage compartment.

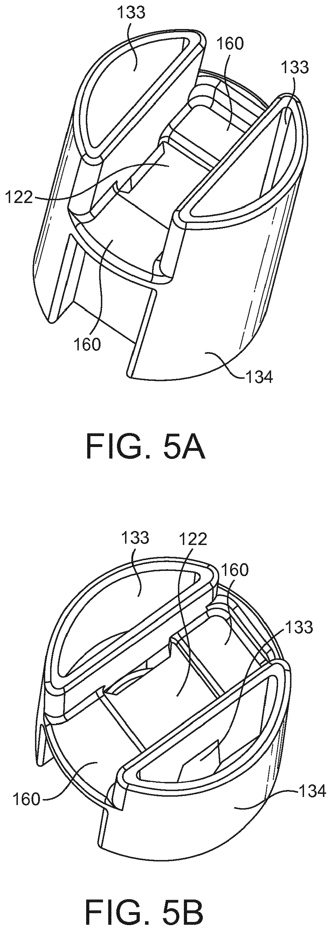

The heater mount 134 and heater assembly 120 are shown in more detail in Figures 5a, 5b, 6a and 6b. Figures 5a and 5b are top perspective views of the heater assembly 120 and heater mount 134 of Figures 4a and 4b. Figures 6a and 6b are bottom views of the heater assembly 120 and heater mount 134 of Figures 4a and 4b. The end cap 138 and capillary material 136 are removed.

Figures 5a and 5b show covering surfaces 160 of the heater mount 134 that cover the first side of the contacts portions 121 of the heater assembly 120, while the mesh heater element 122 is exposed. Liquid channels 133 from the first portion 130 of the liquid storage compartment to the second portion 135 of the liquid storage compartment are defined by vertical walls of the heater mount 134. The same walls also bound the airflow passage 140 as it passes over the heater element 120.

The heater mount is injection moulded and formed from an engineering polymer, such as polyetheretherketone (PEEK) or LCP (liquid crystal polymer).

Figures 6a and 6b show how the heater mount 134 isolates the contact pads 121 from the second portion 135 of the liquid storage compartment but allow the contact pads 121 to be accessible. A wall of the heater mount 134 isolates the contact portions 121 from the liquid in the storage portion. The heater mount 134 also isolates the exposed portion of the contact pads 121 from the air flow passage 140.

The overmoulding of the heater mount 134 on the heater assembly 120 provides a robust component that can be easily handled during assembly of the system without damaging delicate portions of the heater element 134.

The liquid may be inserted into the liquid storage compartment from the bottom end, before the end cap 138 is fixed, or through a filling port (not shown) in the upper storage portion housing, after the end cap 138 is fixed. The liquid storage compartment may be refillable through a filling port.

The liquid storage compartment may then be fixed inside a cartridge housing 105 using a mechanical fixing or using another means, such as adhesive or welding for example. Alternatively the storage compartment may be fixed to or removably coupled to the housing of a control body of an aerosol-generating system.

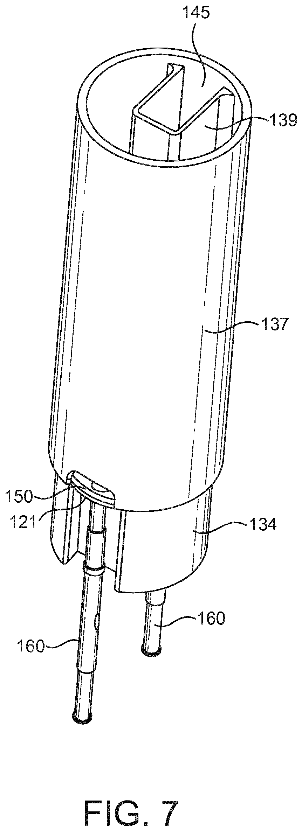

Figure 7 illustrates how electrical contacts in a control body of an aerosol-generating system can be arranged to mate with the exposed contact pads of the heater assembly 120. Only the electrical contacts of the control body 200 are shown. The electrical contacts comprise a pair of spring loaded pins 160 that extend in the slots formed on either side of the heater mount 134 to contact the contact pads 121. With this arrangement the cartridge 100 can be inserted in or joined to the control body 200 by moving the cartridge 100 into contact with the pins in an insertion direction parallel to the longitudinal axis of the pins. When the pins are in contact with the contact pads 121, electrical current can be delivered to the heating element 122. The cartridge 100 may be retained within a control body housing or may be fixed to the control body using a push fitting or snap fitting.

Figure 7 also shows a cut away portion of the upper storage compartment housing 137. It can be seen that an internal wall 139 is used to divide the airflow passage 145 from the liquid 131 with in the liquid storage compartment. Air inlet 150 is also clearly illustrated.

The operation of the system will now be briefly described. The system is first switched on using a switch on the control body 200 (not shown in Figure 1). The system may comprise an airflow sensor in fluid communication with the airflow passage can be puff activated. This means that the control circuitry 220 is configured to supply power to the heating element 122 based on signals from the airflow sensor. When the user wants to inhale aerosol, the user puffs on the mouth end opening 110 of the system. Alternatively the supply of power to the heating element 122 may be based on user actuation of a switch. When power is supplied to the heating element 122, the heating element 122 heats to temperature above a vaporisation temperature of the liquid aerosol-forming substrate 131. The liquid aerosol-forming substrate 131 lose to the heating element 122 is thereby vapourised and escapes into the airflow passage 140. The mixture of air drawn in through the air inlet 150 and the vapour from the heating element 122 is drawn through the airflow passage 140, 145 towards the mouth end opening 110. As it travels through the airflow passage 140, 145 the vapour cools to form an aerosol, which is then drawn into the user's mouth. At the end of the user puff or after a set time period, power to the heating element 122 is cut and the heater cools again before the next puff.

During normal use in this manner, and between user puffs, the system is typically held so that the mouth end of the system is uppermost. This means that the first portion 130 of the liquid storage compartment is above the second portion 135 of the liquid storage compartment, and the heating element is above the capillary material 136 in the second portion 135 of the liquid storage compartment. As liquid in the capillary material 136 close to the heating element 122 is vapourised and escapes into the airflow passage 140, it is replenished by liquid from the first portion 130 of the liquid storage compartment flowing into the capillary material 136 under the influence of gravity. The liquid from the first portion 130 flows through the two enclosed liquid flow paths 133 into the capillary material 136. The capillary material 136 then draws the liquid up to the heating element 122 ready for the next user puff. The direction of travel of the liquid is illustrated by the arrows in Figure 2a.

Although the invention has been described in relation to a system comprising a control body and a separate but connectable cartridge, it should be clear that the arrangement of the heater mount moulded on the heater assembly, and the configuration of the liquid storage compartment, airflow passage and heater assembly could be used in a one-piece aerosol-generating system.

It should also be clear that alternative geometries are possible within the scope of the invention. In particular, the airflow passage may extend through the first portion of the storage compartment in a different manner, such as through a centre of the liquid storage compartment. The cartridge and liquid storage compartment may have a different cross-sectional shape and the heater assembly may have a different shape and configuration.

An aerosol-generating system having the construction described has several advantages. The possibility of liquid leaking into the air flow passage is reduced by the arrangement of the first and second portions of the liquid storage compartment. The possibility of liquid or vapour damaging or corroding the electrical contact portions is significantly reduced by the construction of the heater mount. The construction is robust and inexpensive and results in minimal wastage of liquid aerosol-forming substrate.

## Claims

1. A cartridge (100) for an aerosol-generating system, the cartridge comprising:
a storage compartment containing a liquid aerosol-forming substrate (131), the storage compartment having a first portion (130) and a second portion (135) connected to one another by a liquid channel so that liquid in the first portion can pass to the second portion through the liquid channel;
**characterised in that** the cartridge further comprises:
an air flow passage (140) passing between the first portion and the second portion of the storage compartment;
a fluid permeable aerosol-generating element positioned between the first portion and the second portion of the storage compartment and having a first side and a second side opposing the first side, the first side of the aerosol-generating element forming part of the airflow passage and the second side of the aerosol-generating element being in contact with liquid from the second portion of the storage compartment, so that liquid aerosol-forming substrate in the first portion of the storage compartment can reach the fluid permeable aerosol-generating element only through the second portion of the storage compartment.

2. A cartridge according to claim 1, wherein the first portion of the storage compartment has a larger liquid storage capacity than the second portion of the storage compartment.

3. A cartridge according to claim 1 or claim 2, wherein the second portion of the storage compartment contains a capillary material (136) in contact with the second side of the aerosol-generating element.

4. A cartridge according to any one of the preceding claims, wherein the cartridge comprises a housing (105) having a connection end (115) and a mouth end remote from the connection end, the connection end configured to connect to a control body (200) of an aerosol-generating system, the second side of the aerosol-generating element facing the connection end and the first side of the aerosol-generating element facing the mouth end.

5. A cartridge according to claim 4, wherein the aerosol-generating element is closer to the connection end than to the mouth end opening.

6. A cartridge according to any preceding claim, further comprising pan air inlet (150) and a mouthpiece portion having a mouth end opening (110), the airflow passage extending from the air inlet, between the first portion and the second portion of the storage compartment, to the mouth end opening, wherein the first portion of the storage compartment is positioned between the aerosol-generating element and the mouth end opening.

7. A cartridge according to any one of the preceding claims, wherein the first and second sides of the aerosol-generating element are planar.

8. A cartridge according to any one of the preceding claims wherein the aerosol-generating element comprises a plurality of interstices or apertures extending from the second side to the first side and through which fluid may pass.

9. A cartridge according to any of the preceding claims, wherein the aerosol-generating element is a heating element.

10. A cartridge according to claim 9, comprising a heater assembly (120), the heater assembly comprising the heating element and electrical contact portions, electrically connected to the heating element

11. A cartridge according to claim 10, wherein the contact portions are exposed through a connection end of the cartridge.

12. A cartridge according to claim 10 or 11, wherein the storage compartment comprises a heater mount (134), the heater mount being moulded over the heater assembly.

13. An aerosol-generating system comprising a cartridge in accordance with any one of the preceding claims and a control body (200) connected to the cartridge, the control body configured to control a supply of electrical power to the aerosol-generating element.

14. An aerosol-generating system according to claim 13, the control body comprising a pair of electrical contacts configured to engage electrical contact portions in the cartridge.

15. An aerosol-generating system according to claim 13 or 14, wherein the aerosol-generating system is a handheld aerosol-generating system.

## Patentansprüche

1. Patrone (100) für ein Aerosolerzeugungssystem, die Patrone umfassend:
eine ein flüssiges aerosolbildendes Substrat (131) enthaltende Aufbewahrungskammer, wobei die
Aufbewahrungskammer einen ersten Abschnitt (130) und einen zweiten Abschnitt (135) aufweist, die durch einen Flüssigkeitskanal miteinander verbunden sind, sodass Flüssigkeit in dem ersten Abschnitt durch den Flüssigkeitskanal zu dem zweiten Abschnitt strömen kann;
**dadurch gekennzeichnet, dass** die Patrone ferner umfasst:
einen Luftstromkanal (140), der zwischen dem ersten Abschnitt und dem zweiten Abschnitt der Aufbewahrungskammer verläuft;
ein fluiddurchlässiges aerosolerzeugendes Element, das zwischen dem ersten Abschnitt und dem zweiten Abschnitt der Aufbewahrungskammer positioniert ist und eine erste Seite und eine zweite Seite gegenüber der ersten Seite aufweist, wobei die erste Seite des aerosolerzeugenden Elements einen Teil des Luftstromkanals bildet und die zweite Seite des aerosolerzeugenden Elements in Kontakt mit Flüssigkeit von dem zweiten Abschnitt der Aufbewahrungskammer steht, sodass flüssiges aerosolbildendes Substrat in dem ersten Abschnitt der Aufbewahrungskammer das fluiddurchlässige aerosolerzeugende Element nur durch den zweiten Abschnitt der Aufbewahrungskammer erreichen kann.

2. Patrone nach Anspruch 1, wobei der erste Abschnitt der Aufbewahrungskammer eine größere Flüssigkeitsspeicherkapazität als der zweite Abschnitt der Aufbewahrungskammer aufweist.

3. Patrone nach Anspruch 1 oder Anspruch 2, wobei der zweite Abschnitt der Aufbewahrungskammer ein Kapillarmaterial (136) in Kontakt mit der zweiten Seite des aerosolerzeugenden Elements enthält.

4. Patrone nach einem der vorhergehenden Ansprüche, wobei die Patrone ein Gehäuse (105), aufweisend ein Verbindungsende (115) und ein von dem Verbindungsende entferntes Mundende umfasst, wobei das Verbindungsende zur Verbindung mit einem Regelkörper (200) eines Aerosolerzeugungssystems ausgelegt ist, wobei die zweite Seite des aerosolerzeugenden Elements dem Verbindungsende zugewandt ist und die erste Seite des aerosolerzeugenden Elements dem Mundende zugewandt ist.

5. Patrone nach Anspruch 4, wobei das aerosolerzeugende Element näher an dem Verbindungsende als an der Öffnung des Mundendes ist.

6. Patrone nach einem beliebigen vorhergehenden Anspruch, ferner umfassend einen Lufteinlass (150) und einen eine Öffnung des Mundendes (110) aufweisenden Mundstückabschnitt, wobei sich der Luftstromkanal von dem Lufteinlass zwischen dem ersten Abschnitt und dem zweiten Abschnitt der Aufbewahrungskammer zu der Öffnung des Mundendes erstreckt, wobei der erste Abschnitt der Aufbewahrungskammer zwischen dem aerosolerzeugenden Element und der Öffnung des Mundendes positioniert ist.

7. Patrone nach einem der vorhergehenden Ansprüche, wobei die erste und die zweite Seite des aerosolerzeugenden Elements planar sind.

8. Patrone nach einem der vorhergehenden Ansprüche, wobei das aerosolerzeugende Element eine Vielzahl von Zwischenräumen oder Öffnungen aufweist, die sich von der zweiten Seite zu der ersten Seite erstrecken und durch die Fluid strömen kann.

9. Patrone nach einem beliebigen der vorhergehenden Ansprüche, wobei das aerosolerzeugende Element ein Heizelement ist.

10. Patrone nach Anspruch 9, umfassend eine Heizvorrichtungsbaugruppe (120), wobei die Heizvorrichtungsbaugruppe das Heizelement und elektrische Kontaktabschnitte umfasst, die mit dem Heizelement elektrisch verbunden sind.

11. Patrone nach Anspruch 10, wobei die Kontaktabschnitte durch ein Verbindungsende der Patrone freiliegen.

12. Patrone nach Anspruch 10 oder 11, wobei die Aufbewahrungskammer eine Heizvorrichtungshalterung (134) umfasst, wobei die Heizvorrichtungshalterung über die Heizvorrichtungsbaugruppe formgegossen ist.

13. Aerosolerzeugungssystem, umfassend eine Patrone nach einem der vorhergehenden Ansprüche und einen mit der Patrone verbundenen Regelkörper (200), wobei der Regelkörper zum Regeln einer Zufuhr von elektrischer Energie an das aerosolerzeugende Element ausgelegt ist.

14. Aerosolerzeugungssystem nach Anspruch 13, wobei der Regelkörper ein Paar elektrischer Kontakte umfasst, die zum Eingriff in elektrische Kontaktabschnitte in der Patrone ausgelegt sind.

15. Aerosolerzeugungssystem nach Anspruch 13 oder 14, wobei das Aerosolerzeugungssystem ein handgehaltenes Aerosolerzeugungssystem ist.

## Revendications

1. Cartouche (100) destinée à être utilisée dans un système de génération d'aérosol, la cartouche comprenant :
un compartiment de stockage contenant un substrat formant aérosol liquide (131), le compartiment de stockage ayant une première partie (130) et une deuxième partie (135) raccordées l'une à l'autre par un canal de liquide de sorte que le liquide dans la première partie peut passer vers la deuxième partie à travers le canal de liquide ;
**caractérisé en ce que** la cartouche comprend en outre :
un passage d'écoulement d'air (140) passant entre la première partie et la deuxième partie du compartiment de stockage ;
un élément de génération d'aérosol perméable aux fluides positionné entre la première partie et la deuxième partie du compartiment de stockage et ayant un premier côté et un deuxième côté opposé au premier côté, le premier côté de l'élément de génération d'aérosol faisant partie du passage d'écoulement d'air et le deuxième côté de l'élément de génération d'aérosol étant en contact avec le liquide provenant de la deuxième partie du compartiment de stockage, afin que le substrat formant aérosol liquide dans la première partie du compartiment de stockage puisse atteindre l'élément de génération d'aérosol perméable aux fluides uniquement à travers la deuxième partie du compartiment de stockage.

2. Cartouche selon la revendication 1, dans laquelle la première partie du compartiment de stockage a une plus grande capacité de stockage de liquide que la deuxième partie du compartiment de stockage.

3. Cartouche selon la revendication 1 ou la revendication 2, dans laquelle la deuxième partie du compartiment de stockage contient une matière capillaire (136) en contact avec le deuxième côté de l'élément de génération d'aérosol.

4. Cartouche selon l'une quelconque des revendications précédentes, dans laquelle la cartouche comprend un logement (105) ayant une extrémité de raccordement (115) et une extrémité buccale éloignée de l'extrémité de raccordement, l'extrémité de raccordement étant configurée pour se raccorder à un corps de commande (200) d'un système de génération d'aérosol, le deuxième côté de l'élément de génération d'aérosol faisant face à l'extrémité de raccordement et le premier côté de l'élément de génération d'aérosol faisant face à l'extrémité buccale.

5. Cartouche selon la revendication 4, dans laquelle l'élément de génération d'aérosol est plus proche de l'extrémité de raccordement que de l'ouverture d'extrémité buccale.

6. Cartouche selon l'une quelconque des revendications précédentes, comprenant en outre une entrée d'air (150) et une partie d'embout buccal ayant une ouverture (110) d'extrémité buccale, le passage d'écoulement d'air s'étendant depuis l'entrée d'air, entre la première partie et la deuxième partie du compartiment de stockage, jusqu'à l'ouverture d'extrémité buccale, dans laquelle la première partie du compartiment de stockage est positionnée entre l'élément de génération d'aérosol et l'ouverture d'extrémité buccale.

7. Cartouche selon l'une quelconque des revendications précédentes, dans laquelle les premier et deuxième côtés de l'élément de génération d'aérosol sont plans.

8. Cartouche selon l'une quelconque des revendications précédentes, dans laquelle l'élément de génération d'aérosol comprend une pluralité d'interstices ou d'ouvertures s'étendant du deuxième côté au premier côté et à travers lesquels le fluide peut passer.

9. Cartouche selon l'une quelconque des revendications précédentes, dans laquelle l'élément de génération d'aérosol est un élément de chauffage.

10. Cartouche selon la revendication 9, comprenant un ensemble de chauffage (120), l'ensemble de chauffage comprenant l'élément de chauffage et des parties de contact électrique, raccordées électriquement à l'élément de chauffage.

11. Cartouche selon la revendication 10, dans laquelle les parties de contact sont exposées à travers une extrémité de raccordement de la cartouche.

12. Cartouche selon la revendication 10 ou 11, dans laquelle le compartiment de stockage comprend un support de dispositif de chauffage (134), le support de dispositif de chauffage étant moulé sur l'ensemble de chauffage.

13. Système de génération d'aérosol comprenant une cartouche selon l'une quelconque des revendications précédentes et un corps de commande (200) raccordé à la cartouche, le corps de commande étant configuré pour commander une alimentation en courant électrique vers l'élément de génération d'aérosol.

14. Système de génération d'aérosol selon la revendication 13, le corps de commande comprenant une paire de contacts électriques configurés pour mettre en prise des parties de contact électrique dans la cartouche.

15. Système de génération d'aérosol selon la revendication 13 ou 14, dans lequel le système de génération d'aérosol est un système de génération d'aérosol portatif.
